# EUROPEAN PATENT APPLICATION

(11) **EP 4 714 436 A1**
(43) Date of publication of application: **25.03.2026**
(21) Application number: 23945728.6
(22) Date of filing: 22.12.2023
(51) Int. Cl.: A61K 9/51, A61K 48/00, A61K 47/52, A61K 47/54, A61K 47/59, A61K 31/7105, A61K 39/00, C12N 15/88, A61K 47/30, A61P 35/00, A61P 31/00, A61P 9/00, A61P 1/00, A61P 1/16, A61P 17/18

(54) **MRNA/LIPID-POLYMER HYBRID NANOPARTICLE DELIVERY SYSTEM, AND PREPARATION METHOD THEREFOR AND USE THEREOF**

(30) Priority: 14.07.2023 CN 202310863935
(71) Applicant: Liangzhu Laboratory, Hangzhou, Zhejiang 310000 (CN)
(72) Inventor: KONG, Na, Hangzhou, Zhejiang 310000 (CN); XIAO, Fan, Hangzhou, Zhejiang 310000 (CN)
(74) Representative: Rapisardi, Mariacristina
(86) International application number: PCT/CN2023/140929
(87) International publication number: WO 2025/015850

(57) **Abstract**

An mRNA/lipid-polymer hybrid nanoparticle delivery system, and a preparation method therefor and the use thereof. The delivery system comprises mRNA, cationic molecules, a polymer and modified amphiphilic molecules, wherein the cationic molecules and the negatively charged mRNA form a compound by means of electrostatic interaction, the polymer is used for accommodating the cationic molecule-mRNA compound, and the three form a stable core structure; and the modified amphiphilic molecules are anchored on the surface of the core structure by means of hydrophobic interaction, thereby forming a sphere-like core-shell structure.

## Description

### TECHNICAL FIELD

The present application belongs to the field of biotechnology and, in particular, relates to an mRNA-lipid-polymer hybrid nanoparticles delivery system, a preparation method therefor, and use thereof.

### BACKGROUND

Messenger RNA (mRNA) is transcribed from a template of DNA and carries genetic information for encoding a target protein, which is subsequently translated into a functional protein under the action of a ribosome. mRNA technology that delivers exogenous mRNA into cells to translate the required functional proteins has become a novel therapeutic approach with great potential. However, naked mRNA exhibits extreme instability and immunogenicity in a biological environment. Moreover, since it is negatively charged in nature, the naked mRNA is difficult to cross a cell membrane that is also negatively charged. Therefore, it is necessary to develop a robust delivery system to protect mRNA, slow down mRNA degradation, and enable mRNA to be successfully delivered into the cytoplasm to take effect after the translation into the target protein.

At present, new materials developed for mRNA delivery are mainly focused on lipid nanoparticles (LNPs). Generally, the LNPs mainly include ionizable lipid molecules, cholesterol-based lipid molecules, auxiliary phospholipid molecules, and polyethylene glycol (PEG)-ylated lipid molecules. The ionizable lipid molecules that are positively charged form complexes with negatively charged mRNA molecules through electrostatic interaction in an acidic buffer solution and facilitate the endosomal escape of mRNA molecules in cells. The cholesterol-based lipid molecules are used to fill gaps between lipids, thereby enhancing the stability of the nanoparticles. The auxiliary phospholipid molecules can regulate the membrane fluidity of the nanoparticles to improve the final performance of the nanoparticles. The polyethylene glycolated lipid molecules are used to reduce the interaction between the nanoparticles and biological macromolecules *in vivo,* thereby increasing the blood circulation and half-life of the nanoparticles. However, the current mRNA delivery system based on LNPs still has several major problems. (1) The composition of LNPs is relatively complex. Apart from the functional mRNA component, at least four other components are required, and each component has a decisive impact on the final delivery effect of mRNA. Therefore, in the development of the LNP-based delivery system, a large amount of financial, material, and human resources is required to optimize the formulation of LNPs. (2) Various lipid molecules in LNPs, such as cholesterol and phospholipids, have relatively small molecular weights and high fluidity. Thus, the overall structure of the prepared LNPs is low in stability, and the half-life of the LNPs during *in vivo* blood circulation is relatively short, making it difficult for the LNPs to achieve the expected effects. (3) The core components of LNPs, including lipid structures such as the ionizable lipid molecules and the auxiliary phospholipid molecules, are relatively difficult to synthesize and scale up for production.

Therefore, there is still an urgent need to develop a novel mRNA delivery system with fewer key components and higher structural stability, so as to improve the translation and expression performance of mRNA and ultimately promote the application and development of mRNA technology.

### SUMMARY

An object of the present application is to disclose an mRNA-lipid-polymer hybrid nanoparticles (LPNPs) delivery system.

A second object of the present application is to disclose a preparation method for the preceding delivery system.

A third object of the present application is to disclose use of the preceding delivery system.

The objects of the present application are achieved by the technical solutions below.

In a first aspect, the present application provides an mRNA-lipid-polymer hybrid nanoparticles delivery system (mRNA-LPNPs delivery system).

An mRNA-lipid-polymer hybrid nanoparticles delivery system, wherein components of the delivery system include mRNA, a cationic molecule, a polymer, and a modified amphiphilic molecule; wherein the cationic molecule and negatively charged mRNA form a complex through electrostatic interaction, the polymer is used to encapsulate the cationic molecule-mRNA complex to form a stable core structure, and the modified amphiphilic molecule is anchored to a surface of the core structure through hydrophobic interaction to form a quasi-spherical core-shell structure.

In the mRNA-LPNPs delivery system according to the preceding technical solution, mRNA may theoretically be any type of mRNA. For example, mRNA includes, but is not limited to, self-reporter gene mRNA, genome editing mRNA, antigen mRNA, tumor suppressor gene mRNA, interleukin mRNA, chimeric antigen receptor mRNA, or other functional mRNA.

In the mRNA-LPNPs delivery system according to the preceding technical solution, the self-reporter gene mRNA includes, but is not limited to, GFP, eGFP, Fluc, mCherry, beta gal or renilla Luc.

The genome editing mRNA includes, but is not limited to, Cas9, Cas9 Nickase, or Cre recombinase.

The antigen mRNA includes, but is not limited to, OVA.

The tumor suppressor gene mRNA includes, but is not limited to, p53 or PTEN.

The interleukin mRNA includes, but is not limited to, IL-10, IL-12, or IL-6.

In the mRNA-LPNPs delivery system according to the preceding technical solution, for example, the cationic molecule includes, but is not limited to, ammonia derivatives, ammonium salts and derivatives thereof, positively charged amphiphilic lipid-like compounds, polyamide and derivatives thereof, or polyethyleneimine and derivatives thereof.

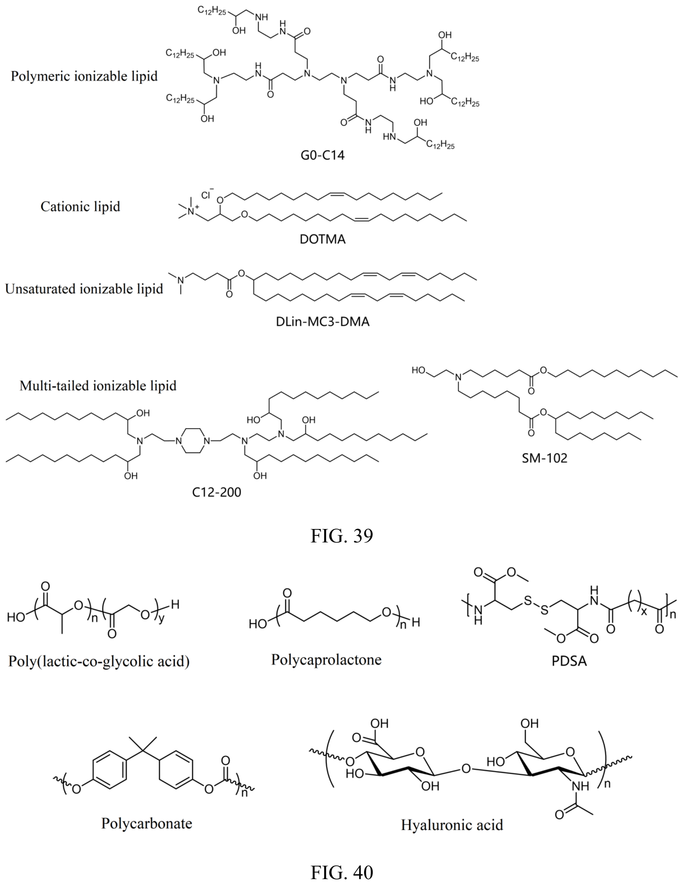

FIG. 39 shows several types of representative cationic molecules among the preceding cationic molecules: permanently positively charged cationic lipids (such as DOTAP, DOTMA, or DMRIE), unsaturated ionizable lipids containing double bonds (such as Dlin-MC3-DMA), multi-tailed ionizable lipids (such as C12-200), and polymeric ionizable lipids (such as a polyamide dendrimer PAMAM or polyethyleneimine PEI).

In the mRNA-LPNPs delivery system according to the preceding technical solution, for example, the polymer includes, but is not limited to, aliphatic polyester polymers and derivatives thereof (such as polylactic acid, polycaprolactone, or poly(lactic-co-glycolic acid)), polycarbonates and derivatives thereof, polysaccharides and derivatives thereof, polyamino acids and derivatives thereof, polymeric polyols and derivatives thereof, or polyacrylic acids and derivatives thereof. FIG. 40 shows several types of representative polymers among the preceding polymer molecules.

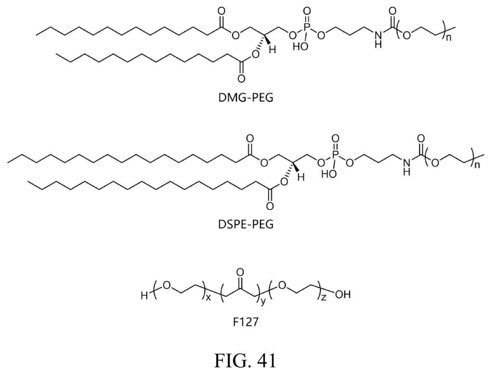

In the mRNA-LPNPs delivery system according to the preceding technical solution, for example, the modified amphiphilic molecule includes, but are not limited to, a polyethylene glycolated amphiphilic molecule (such as DSPE-PEG, DMG-PEG, F127), an amphiphilic surfactant (such as CO-520), an amphiphilic protein (such as albumin), and other modified molecules (such as mannitol, modified chitosan, dextran, carboxydextran, tetraethyl orthosilicate, polyacrylic acid, KH560, KH550, diethylenetriaminepentaacetic acid, meglumine, arginine, polyglutamic acid, or polypeptides). FIG. 41 shows several types of representative molecules among the preceding modified amphiphilic molecules.

In a second aspect, the present application provides a preparation method for the mRNA-LPNPs delivery system of the first aspect. The preparation method includes the following steps:
(a) preparing a solution of a cationic molecule;
(b) forming an electrostatic complex of mRNA and the cationic molecule;
(c) sequentially adding a polymer and a modified amphiphilic molecule to an mRNA/cationic molecule complex formed above; and
(d) obtaining stable mRNA-encapsulated nanoparticles by a self-assembly method, that is, the mRNA-lipid-polymer hybrid nanoparticles delivery system delivery system.

The preparation method for the mRNA-LPNPs delivery system according to the preceding technical solution includes specific steps of:
(a1) dissolving a cationic molecule in a solvent to prepare a solution containing the cationic molecule and having a concentration of 0.5-5.0 mg/mL;
where the cationic molecule include, but is not limited to, at least one of ammonia derivatives, ammonium salts and derivatives thereof, positively charged amphiphilic lipid-like compounds, polyamide and derivatives thereof, or polyethyleneimine and derivatives thereof; and
the solvent includes, but is not limited to, at least one of methanol, ethanol, ethylene glycol, diethylene glycol, DMF, benzyl alcohol, acetone, dichloromethane, or trichloromethane;
(b1) mixing a solution containing mRNA with the solution containing the cationic molecule uniformly, and performing a reaction for a period to obtain an mRNA/cationic molecule electrostatic complex;
where the reaction is performed for a period of 5s to 30 min and at a temperature of 0°C to 37°C;
a concentration of mRNA in the solution containing mRNA is 0.1-10 mg/mL;
a mass ratio of mRNA to the cationic molecule is 1:1 to 1:100;
mRNA may theoretically be any type of mRNA, and for example, mRNA includes, but is not limited to, self-reporter gene mRNA, genome editing mRNA, antigen mRNA, tumor suppressor gene mRNA, interleukin mRNA, chimeric antigen receptor mRNA, or other functional mRNA;
the self-reporter gene mRNA includes, but is not limited to, one of GFP, eGFP, Fluc, mCherry, beta gal, or renilla Luc;
the genome editing mRNA includes, but is not limited to, one of Cas9, Cas9 Nickase, or Cre recombinase;
the antigen mRNA includes, but is not limited to, OVA;
the tumor suppressor gene mRNA includes, but is not limited to, one of p53 or PTEN; and
the interleukin mRNA includes, but is not limited to, one of IL-10, IL-12, or IL-6;
(c1) mixing the mRNA/cationic molecule electrostatic complex with a polymer solution uniformly;
where a reaction is performed for a period of 5s to 30 min and at a temperature of 0°C to 37°C;
a concentration of the polymer molecule in a solution containing the polymer molecule is 0.1-20 mg/mL; and
the polymer molecule includes, but is not limited to, at least one of aliphatic polyester polymers and derivatives thereof (such as polylactic acid, polycaprolactone, or poly(lactic-co-glycolic acid)), polycarbonates and derivatives thereof, polysaccharides and derivatives thereof, polyamino acids and derivatives thereof, polymeric polyols and derivatives thereof, or polyacrylic acids and derivatives thereof;
(d1) adding a solution of the modified amphiphilic molecules into an mRNA/cationic molecule/polymer mixture solution and mixing the solutions uniformly;
where a concentration of the modified amphiphilic molecule in the solution of the modified amphiphilic molecule is 0.1-20 mg/mL; and
the modified amphiphilic molecule include, but is not limited to, at least one of polyethylene glycolated amphiphilic molecules (such as DSPE-PEG, DMG-PEG, or F127), an amphiphilic surfactant (such as CO-520), amphiphilic proteins (such as albumin), or other modified molecules (such as mannitol, modified chitosan, dextran, carboxydextran, tetraethyl orthosilicate, polyacrylic acid, KH560, KH550, diethylenetriaminepentaacetic acid, meglumine, arginine, polyglutamic acid, or polypeptides);
(e1) adding an mRNA/cationic molecule/polymer/modified amphiphilic molecule mixture solution into an acidic aqueous phase solution and performing a reaction for a period to obtain a particle of the mRNA-LPNPs nano-delivery system;
where the acidic solution has a pH range of 1-6;
the reaction is performed for a period of 30 s to 30 min and at a temperature of 0°C to 37°C;
the acidic buffer solution includes, but is not limited to, at least one of citric acid/citrate, acetic acid/acetate, hydrochloric acid/hydrochloride, sulfuric acid/sulfate, or nitric acid/nitrate; and
in steps of (b1), (c1), (d1), and (e1), a solution mixing manner includes, but is not limited to, at least one of dropwise addition, stirring, standing, a microfluidic device, a peristaltic pump, or a microinjection pump.

The preparation method for the mRNA-LPNPs delivery system according to the preceding technical solution adopts the microfluidic device and includes steps of (a1), (b1), and (c1);
(d1) adding the solution of the modified amphiphilic molecule into the mRNA/cationic molecule/polymer mixture solution prepared in (c1) and mixing the solutions uniformly to obtain DMF organic phases;
(e1) preparing an aqueous phase solution; and
(f1) under microfluidic conditions, preparing mRNA-LPNPs with a flowrate of the DMF organic phases being 1-5 mL/min and a flowrate of the aqueous phase solution being 5-25 mL/min.

An mRNA-LPNPs delivery system is prepared by the preparation method according to the preceding technical solution.

In the mRNA-LPNPs delivery system according to the preceding technical solution, encapsulation efficiency of mRNA in the mRNA-LPNPs delivery system is ≥ 50%, preferably ≥ 60%, more preferably ≥ 70%, more preferably ≥ 80%, more preferably ≥ 90%, and most preferably ≥ 95% (for example, 99%).

In the mRNA-LPNPs delivery system according to the preceding technical solution, a mass ratio of mRNA to other molecules in the mRNA-LPNPs delivery system is 1:100 to 10:1.

In the mRNA-LPNPs delivery system according to the preceding technical solution, the mRNA-LPNPs delivery system has a two-dimensional, three-dimensional, or layered structure; the particle of the nano-delivery system is a three-dimensional nanocomposite material with an mRNA-cationic molecule-polymer core coated with a shell of the modified amphiphilic molecules; and a particle size of the particle of the nano-delivery system is 10-500 nm, preferably 20-250 nm, more preferably 30-200 nm, more preferably 70-130 nm, more preferably 100-125 nm, and most preferably 110-125 nm.

In the mRNA-LPNPs delivery system according to the preceding technical solution, based on a total number of mRNA-LPNPs delivery systems, 80% (preferably 85%, more preferably 90%, more preferably 95%, and most preferably ≥ 99%) of the delivery systems have particle sizes within ±15% (preferably ±10% and more preferably ±1%) of a D50 range of the nanocomposite material.

In the mRNA-LPNPs delivery system according to the preceding technical solution, the mRNA-LPNPs delivery system is stably dispersible in an aqueous solution and 0.9% normal saline.

In the mRNA-LPNPs delivery system according to the preceding technical solution, when the mRNA-LPNPs delivery system is stored in water or 0.9% normal saline at 4°C, 25°C, 37°C, -20°C, or -80°C for 5-20 days, preferably 20-40 days, and more preferably 40-60 days, an average particle size of the particle of the nano-delivery system changes by ≤ 20%, preferably ≤ 10%, more preferably ≤ 5%, and most preferably ≤ 3%.

In the mRNA-LPNPs delivery system according to the preceding technical solution, cell transfection efficiency of mRNA in the mRNA-LPNPs delivery system is ≥ 50%, preferably ≥ 60%, more preferably ≥ 70%, more preferably ≥ 80%, more preferably ≥ 90%, and most preferably ≥ 95% (for example, 99%).

In a third aspect, the present application provides use of the mRNA-LPNPs delivery system of the first aspect, where the mRNA-LPNPs delivery system is used for preparation of a tumor therapeutic drug, preparation of a tumor vaccine, preparation of a tumor-targeting drug, preparation of an *in vitro* or *in vivo* tumor diagnostic material, preparation of an inflammation-related disease treatment, preparation of an infectious disease treatment, preparation of infectious disease prevention, preparation of a wound-healing treatment, preparation of a cardiovascular and cerebrovascular chronic disease treatment, preparation of an organ fibrosis treatment, preparation of a viral infection treatment, preparation of viral infection prevention, preparation of an *in vitro* or *in vivo* gene-editing therapy, preparation of a non-alcoholic fatty liver treatment, preparation of an intestinal disease treatment, preparation of a neoantigen vaccine, preparation of cell differentiation induction, preparation of protein-based cosmetics, preparation of a protein defect-related disease treatment, preparation of aging repair, preparation of anti-aging, or preparation of a drug carrier.

In the technical solution, the obtained mRNA-LPNPs delivery system has different uses depending on different functions of mRNA delivered.

In a fourth aspect, the present application provides a product including the mRNA-LPNPs delivery system according to any one of the preceding technical solutions.

The present application has the beneficial effects described below.
1. The components of the LPNP delivery system of the present application for delivering mRNA include mRNA, the cationic molecule, the polymer, and the modified amphiphilic molecule. The introduction of the polymer with a larger molecular weight reduces the internal fluidity of nanoparticles and stabilizes the mRNA-cationic molecule complex, thereby reducing key components of an mRNA delivery system and achieving higher mRNA transfection efficiency.
2. The preparation method for the mRNA-LPNPs delivery system of the present application adopts a stepwise self-assembly method to obtain a series of nanomaterials with an excellent mRNA transfection capability. The nanomaterials have suitable particle sizes, good stability, good water dispersibility, excellent biocompatibility, a stable structure, a small number of material components, high mRNA transfection efficiency, and simple and low-cost raw material production.
3. The preparation method for the mRNA-LPNPs delivery system of the present application has the advantages of environmental friendliness, safety and reliability, simple process, low cost, high yield, easy quality control, and ease of large-scale production.
4. The mRNA-LPNPs delivery system of the present application has high mRNA encapsulation efficiency and can protect the structure of mRNA, reduce the enzymatic degradation rate of mRNA, and lower immunogenicity.
5. The mRNA-LPNPs delivery system of the present application can improve the adaptability of mRNA to temperature storage. Compared with naked mRNA, the nanomaterials of the mRNA-LPNPs delivery system can be stored longer at 4°C, 25°C, 37°C, -20°C, and -80°C.
6. Compared with a traditional LNP delivery system, the mRNA-LPNPs delivery system of the present application has enhanced stability in the overall structure due to the introduction of the polymer with the larger molecular weight and thus can reduce the components of the delivery system and lower the difficulty and cost of large-scale production of raw materials, providing a new platform for efficient mRNA delivery.

### BRIEF DESCRIPTION OF DRAWINGS

FIG. 1 shows the characterization results of the size and morphology of mRNA/G0-C 14 LPNPs.
FIG. 2 shows the characterization of mRNA encapsulation efficiency of mRNA/G0-C14 LPNPs.
FIG. 3 shows variations of sizes of mRNA/G0-C14 LPNPs with time during storage in 1× PBS at 4°C.
FIG. 4 shows GFP fluorescence images of A549 cells co-incubated with mRNA/G0-C14 LPNPs (1.0 ng/µL mRNA) for 24 h.
FIG. 5 shows luciferase expression levels at different time points after intramuscular injection of mRNA/G0-C14 LPNPs (50 ng/µL mRNA, 50 µL).
FIG. 6 shows the characterization results of the size and morphology of mRNA/DOTMA LPNPs.
FIG. 7 shows the characterization of mRNA encapsulation efficiency of mRNA/DOTMA LPNPs.
FIG. 8 shows variations of sizes of mRNA/DOTMA LPNPs with time during storage in 1× PBS at 4°C.
FIG. 9 shows GFP fluorescence images of A549 cells co-incubated with mRNA/DOTMA LPNPs (1.0 ng/µL mRNA) for 24 h.
FIG. 10 shows luciferase expression at different time points after intramuscular injection of mRNA/DOTMA LPNPs (50 ng/µL mRNA, 50 µL).
FIG. 11 shows the characterization results of the size and morphology of mRNA/MC3 LPNPs.
FIG. 12 shows the characterization of mRNA encapsulation efficiency of mRNA/MC3 LPNPs.
FIG. 13 shows variations of sizes of mRNA/MC3 LPNPs with time during storage in 1× PBS at 4°C.
FIG. 14 shows GFP fluorescence images of A549 cells co-incubated with mRNA/MC3 LPNPs (1.0 ng/µL mRNA) for 24 h.
FIG. 15 shows luciferase expression levels at different time points after intramuscular injection of mRNA/MC3 LPNPs (50 ng/µL mRNA, 50 µL).
FIG. 16 shows the characterization results of the size and morphology of mRNA/C12-200 LPNPs.
FIG. 17 shows the characterization of mRNA encapsulation efficiency of mRNA/C12-200 LPNPs.
FIG. 18 shows variations of sizes of mRNA/C12-200 LPNPs with time during storage in 1× PBS at 4°C.
FIG. 19 shows GFP fluorescence images of A549 cells co-incubated with mRNA/C12-200 LPNPs (1.0 ng/µL mRNA) for 24 h.
FIG. 20 shows luciferase expression levels at different time points after intramuscular injection of mRNA/C12-200 LPNPs (50 ng/µL mRNA, 50 µL).
FIG. 21 shows the characterization results of the size and morphology of mRNA/SM-102 LPNPs.
FIG. 22 shows the characterization of mRNA encapsulation efficiency of mRNA/SM-102 LPNPs.
FIG. 23 shows variations of sizes of mRNA/SM-102 LPNPs with time during storage in 1× PBS at 4°C.
FIG. 24 shows GFP fluorescence images of A549 cells co-incubated with mRNA/SM-102 LPNPs (1.0 ng/µL mRNA) for 24 h.
FIG. 25 shows luciferase expression levels at different time points after intramuscular injection of mRNA/SM-102 LPNPs (50 ng/µL mRNA, 50 µL).
FIG. 26 shows the size, stability, and encapsulation efficiency of mRNA PDSA LPNPs.
FIG. 27 shows the characterization of an eGFP expression capability of mRNA PDSA LPNPs in Hep3B cells.
FIG. 28 shows the cell viability of Hep3B cells co-incubated with mRNA PDSA LPNPs having different concentrations.
FIG. 29 shows the characterization of the morphology, size, and stability and the encapsulation efficiency of mRNA HA LPNPs.
FIG. 30 shows the characterization of an eGFP expression capability of mRNA HA LPNPs in H1299 cells.
FIG. 31 shows luciferase expression levels at different time points after intramuscular injection of mRNA HA LPNPs (50 ng/µL mRNA, 50 µL).
FIG. 32 is a schematic diagram of operations of microfluidic preparation of mRNA/G0-C14 LPNPs.
FIG. 33 shows the size and morphology characterization of mRNA/G0-C14 LPNPs prepared through microfluidics.
FIG. 34 shows the characterization of mRNA encapsulation efficiency of mRNA/G0-C14 LPNPs prepared through microfluidics.
FIG. 35 shows the *in vitro* cytotoxicity of mRNA/G0-C14 LPNPs having different concentrations.
FIG. 36 shows bioluminescence levels of A549 cells transfected with mRNA/G0-C14 LPNPs having different concentrations.
FIG. 37 shows luciferase expression levels at different time points after intramuscular injection of mRNA/G0-C14 LPNPs (50 ng/µL mRNA, 50 µL).
FIG. 38 is a schematic diagram of a preparation process of an mRNA-LPNPs delivery system of the present application.
FIG. 39 shows several types of representative cationic molecules among cationic molecules.
FIG. 40 shows several types of representative polymers among polymer molecules.
FIG. 41 shows several types of representative molecules among modified amphiphilic molecules.

### DETAILED DESCRIPTION

To facilitate understanding of the technical solutions of the present application, an mRNA-LPNPs delivery system, a preparation method therefor, and use thereof are further described below in conjunction with embodiments.

In view of drawbacks of an existing LNP system for mRNA delivery, the inventors of the present application provide the technical solutions of the present application through research and practice. The technical solutions of the present application are clearly and completely described below. Apparently, the embodiments described below are part, not all, of embodiments of the present application. Based on the embodiments of the present application, all other embodiments obtained by those of ordinary skill in the art without creative work are within the scope of the present application.

The key general testing methods and equipment in the present application are described below.

### (1) Dynamic light scattering (DLS) characterization

A solution of mRNA nanoparticles was measured using Zetasizer of Malvern Panalytical at a scattering angle of 90°. A total concentration of a sample was maintained within 0.1-5.0 mg/mL.

### (2) Transmission electron microscopy (TEM) imaging

Testing instrument: JEOL-2100 Transmission Electron Microscope (TEM); testing conditions: 200 KV, 101 µA; and the nanoparticles to be tested were dispersed in water for the measurement.

### (3) Agarose gel electrophoresis

All samples to be tested were subjected to electrophoresis in 1% agarose gel under the electrophoresis conditions of 100 V and 30 min, with a 1× TAE buffer as an electrophoresis buffer.

### (4) Cytotoxicity test

Cells were inoculated in a 96-well plate at a density of 1×10⁴ cells/well and cultured for 24 h.

An old culture medium was replaced with a complete medium containing mRNA nanoparticles and incubated for 24 h.

10 µL of MTT PBS solution (5 mg/mL) was added to each well and incubated for 4 h.

The culture medium was carefully removed, and 100 µL of dimethyl sulfoxide was added to each well.

The absorbance was measured at a wavelength of 490 nm using a microplate reader, and cell viability was calculated.

### (5) Cell level expression experiment of GFP mRNA

Cells were inoculated in a 96-well plate at a density of approximately 1×10⁴ cells/well and cultured for 24 h. An old culture medium was replaced with a complete medium containing mRNA nanoparticles and cultured for 24 h.

Under blue light excitation, the fluorescence imaging of GFP mRNA was observed using a fluorescence microscope.

### (6) Animal level expression experiment of luciferase mRNA

Hind legs of mice 4-6 weeks old were depilated, followed by intramuscular injection of mRNA nanoparticles. Bioluminescence imaging results of the mice were separately observed at different time points.

The mice were intraperitoneally injected with sodium luciferin (150 mg/kg), and bioluminescence imaging was performed 5 min later.

An aspect of embodiments of the present application provides a preparation method for an LPNP delivery system for mRNA delivery. The technical solutions of the present application are further described in detail below in conjunction with preferred embodiments and the figures. This embodiment is implemented on the premise of the technical solutions of the present application and provides detailed implementation manners and specific operation processes. However, the scope of the present application is not limited to the examples below.

Unless otherwise specified, experimental materials used in the examples below can all be purchased from conventional biochemical reagent companies.

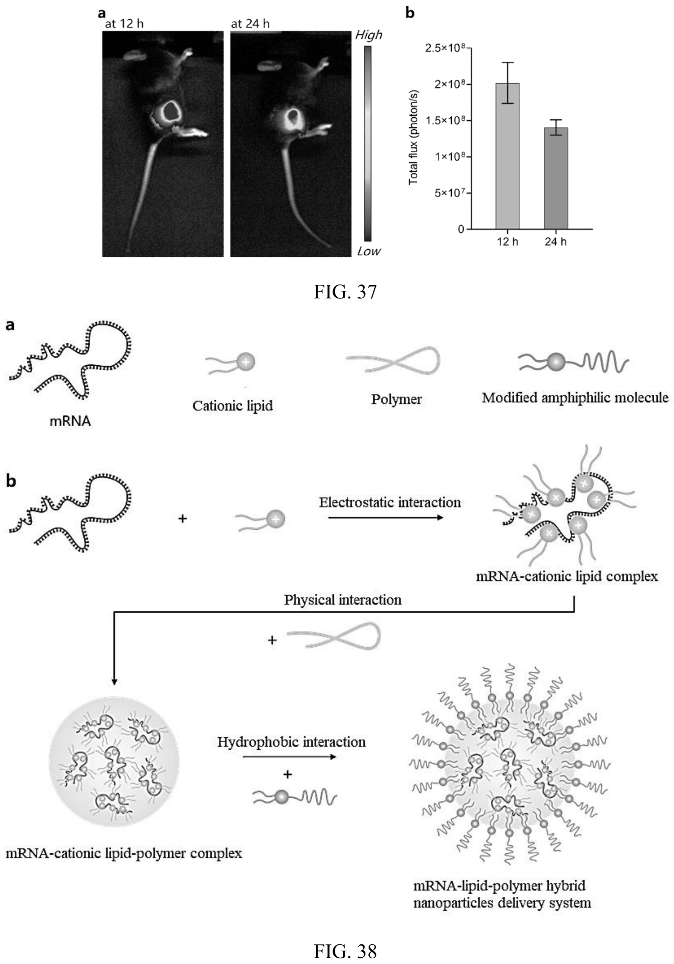

A preparation process of the present application is shown in FIG. 38, where several examples are presented below representatively, which mainly include three types of polymers (PLGA, PDSA, HA) and five types of cationic lipids (G0-C14, DOTMA, MC3, C12-200, and SM-102), forming a total of seven types of mRNA-LPNPs, and one representative example of microfluidic preparation of mRNA-LPNPs.

### Example 1: Preparation of polymer PLGA-based mRNA-LPNPs

With PLGA (having a molecular weight of 30,000-60,000) as the polymer and DSPE-PEG₂ₖ (CAS No.: 147867-65-0) as the modified amphiphilic molecule in the mRNA-LPNPs delivery system, types of cationic lipids/ionizable lipids were changed so that several different mRNA-LPNPs delivery systems were prepared.

### Example 1.1: mRNA/G0-C14 LPNPs (Polymer-based ionizable lipid-LPNPs)

(1) Synthesis of the cationic molecule G0-C14: 1 mmol of a PAMAM dendrimer (ethylenediamine core, Generation G0) methanol solution (CAS No.: 155773-72-1) and 7 mmol of 1,2-epoxytetradecane (CAS No.: 3234-28-4) were added to a 20 mL reaction flask to form a mixed reaction solution. The mixed reaction solution was distilled under negative pressure to remove the methanol solution. Subsequently, under argon protection, the reaction was carried out at 90°C and a stirring speed of 800 rpm for 48 h.. After the reaction was completed, the reaction product was diluted with dichloromethane, and separated and purified through silica gel column chromatography to obtain the cationic lipid G0-C14.
(2) Preparation of key component stock solutions: G0-C14, PLGA, and DSPE-PEG were fully dissolved in N,N-dimethylformamide (DMF) to obtain the corresponding component stock solutions: G0-C14 (2.5 mg/mL), PLGA (5.0 mg/mL), and DSPE-PEG (20 mg/mL).
(3) 50 mM and 10 mM citrate buffers (pH = 4.0) were prepared and treated with DEPC to be made nuclease-free.
(4) Preparation of an mRNA/G0-C14 complex: 10 µL of mRNA (1 µg/µL) was taken, added with 2.5 µL of citrate buffer (50 mM), mixed uniformly, quickly added to 50 µL of G0-C14 DMF solution under vortexing, and continued being vortexed for 20 s to obtain the mRNA/G0-C14 complex.
(5) 50 µL of PLGA DMF solution was sequentially added to the above mRNA/G0-C14 complex and mixed uniformly for 10 s.
(6) 25 µL of DSPE-PEG DMF solution was further added to the above solution and mixed uniformly for 10 s to obtain solution A.
(7) Under vortexing, solution A was quickly added to 625 µL of citrate buffer (10 mM) and continued being vortexed for 50 s.
(8) Under vortexing, 625 µL of 1× PBS was slowly added dropwise to the above mixed solution.
(9) An organic solvent was removed using an ultrafiltration centrifugation method (molecular weight cutoff: 100 kDa), which was repeated three times under the centrifugation conditions of 3000 g and 8 min.
(10) Finally, the volume was adjusted to 500 µL with 1×PBS to obtain the mRNA/G0-C14 LPNPs (mRNA concentration: 20 ng/µL).

### Results and performance characterization

### 1. Size and morphology of mRNA/G0-C14 LPNPs

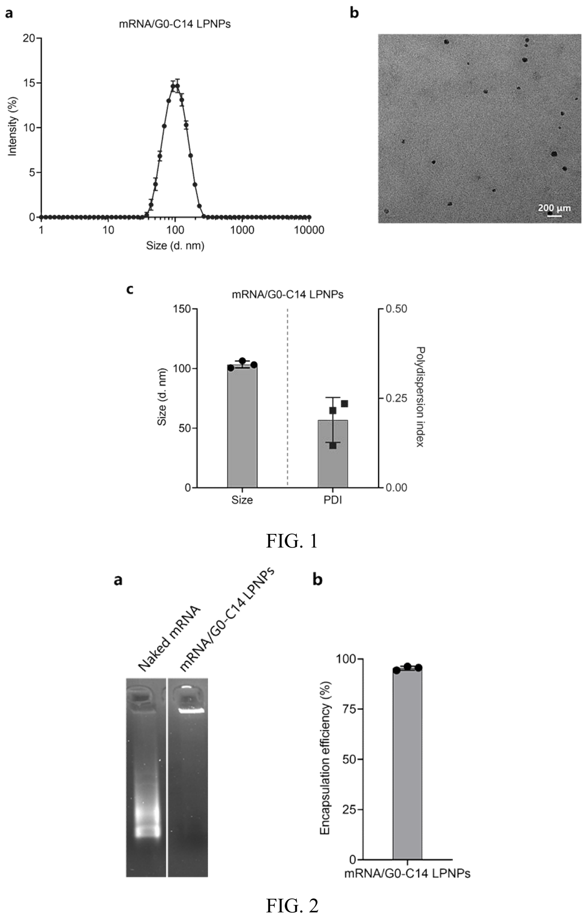

FIG. 1 shows the characterization results of the size and morphology of mRNA/G0-C14 LPNPs. FIG. 1-a shows DLS size distribution. The DLS results show uniform size distribution (d.size = 103 ± 3 nm and PDI = 0.19 ± 0.06). FIG. 1-b shows a TEM image showing that the prepared mRNA nanoparticles have a quasi-spherical structure. FIG. 1-c shows the sizes and PDI distribution of different preparation batches.

### 2. mRNA encapsulation efficiency of mRNA/G0-C14 LPNPs

FIG. 2 shows the characterization of mRNA encapsulation efficiency of mRNA/G0-C14 LPNPs. FIG. 2-a shows a representative agarose gel electrophoresis image of mRNA/G0-C14 LPNPs. FIG. 2-b shows the mRNA encapsulation efficiency of mRNA/G0-C14 LPNPs prepared in different preparation batches. FIG. 2 shows that the prepared mRNA/G0-C14 LPNPs can achieve an mRNA encapsulation efficiency of approximately 95%.

### 3. Size stability of mRNA/G0-C14 LPNPs

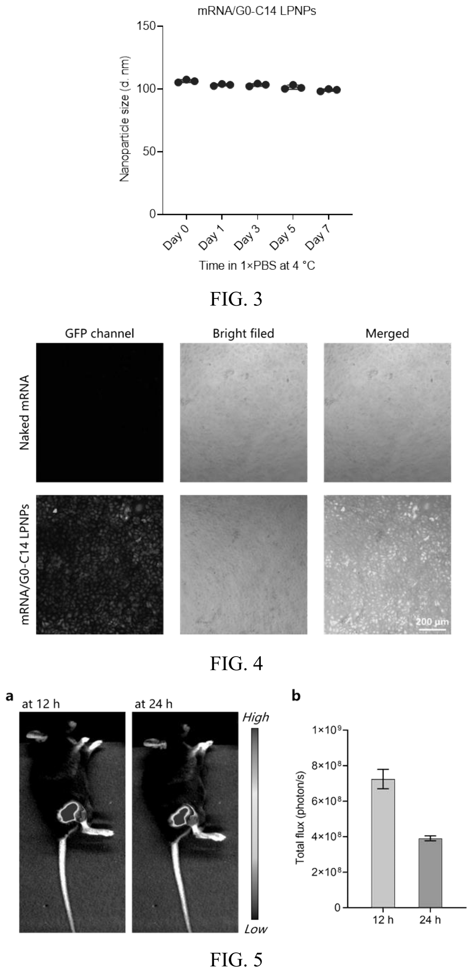

FIG. 3 shows variations of sizes of mRNA/G0-C14 LPNPs with time during storage in 1× PBS at 4°C. FIG. 3 shows that the prepared mRNA/G0-C14 LPNPs have good size stability in 1× PBS at 4°C.

### 4. GFP expression of mRNA/G0-C14 LPNPs at the cell level

FIG. 4 shows GFP fluorescence images of A549 cells co-incubated with mRNA/G0-C14 LPNPs (1.0 ng/µL mRNA) for 24 h. FIG. 4 shows that the mRNA/G0-C14 LPNPs prepared with GFP mRNA as a model exhibit efficient protein expression performance at the cell level.

### 5. Luciferase expression of mRNA/G0-C14 LPNPs at the animal level

FIG. 5 shows luciferase expression levels at different time points after intramuscular injection of mRNA/G0-C14 LPNPs (50 ng/µL mRNA, 50 µL). FIG. 5-a shows the bioluminescence images of a mouse at different time points after intramuscular injection of mRNA/G0-C14 LPNPs. FIG. 5-b shows a quantitative analysis of bioluminescence of mice. FIG. 5 shows that the mRNA/G0-C14 LPNPs prepared with luciferase mRNA as a model also exhibit efficient protein expression performance at the animal level.

### Example 1.2: mRNA/DOTMA LPNPs (Permanently positively charged cationic lipid-LPNPs)

This example differs from Example 1.1 only in that the cationic lipid in Example 1.1 was replaced with DOTMA. The rest of the preparation process remained the same to obtain mRNA/DOTMA LPNPs, which were also subjected to the following structure and performance characterization.

### 1. Size and morphology of mRNA/DOTMA LPNPs

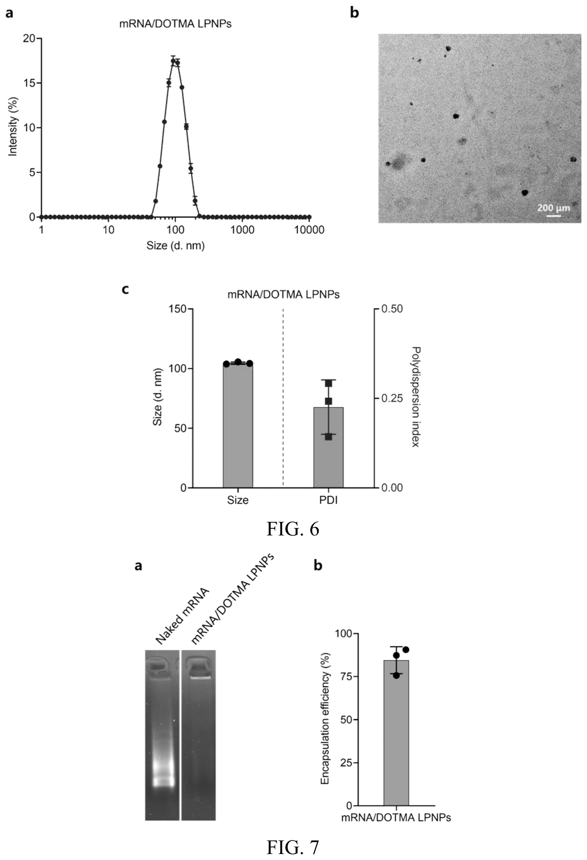

FIG. 6 shows the characterization results of the size and morphology of mRNA/DOTMA LPNPs. FIG. 6-a shows DLS size distribution. The DLS results show uniform size distribution (d.size = 105 ± 1 nm and PDI = 0.23 ± 0.08). FIG. 6-b shows a TEM image showing that the prepared mRNA nanoparticles have a quasi-spherical structure. FIG. 6-c shows the sizes and PDI distribution of different preparation batches.

### 2. mRNA encapsulation efficiency of mRNA/DOTMA LPNPs

FIG. 7 shows the characterization of mRNA encapsulation efficiency of mRNA/DOTMA LPNPs. FIG. 7-a shows a representative agarose gel electrophoresis image of mRNA/DOTMA LPNPs. FIG. 7-b shows the mRNA encapsulation efficiency of mRNA/DOTMA LPNPs prepared in different preparation batches. FIG. 7 shows that the prepared mRNA/DOTMA LPNPs can achieve an mRNA encapsulation efficiency of approximately 85%.

### 3. Size stability of mRNA/DOTMA LPNPs

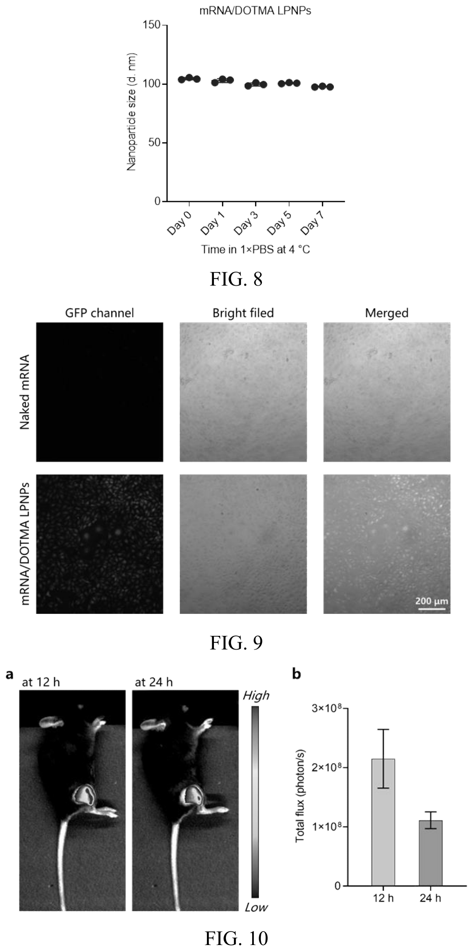

FIG. 8 shows variations of sizes of mRNA/DOTMA LPNPs with time during storage in 1× PBS at 4°C. FIG. 8 shows that the prepared mRNA/DOTMA LPNPs have good size stability in 1× PBS at 4°C.

### 4. GFP expression of mRNA/DOTMA LPNPs at the cell level

FIG. 9 shows GFP fluorescence images of A549 cells co-incubated with mRNA/DOTMA LPNPs (1.0 ng/µL mRNA) for 24 h. FIG. 9 shows that the mRNA/DOTMA LPNPs prepared with GFP mRNA as a model exhibit efficient protein expression performance at the cell level.

### 5. Luciferase expression of mRNA/DOTMA LPNPs at the animal level

FIG. 10 shows luciferase expression at different time points after intramuscular injection of mRNA/DOTMA LPNPs (50 ng/µL mRNA, 50 µL). FIG. 10-a shows the bioluminescence images of a mouse at different time points after intramuscular injection of mRNA/DOTMA LPNPs. FIG. 10-b shows a quantitative analysis of bioluminescence of mice. FIG. 10 shows that the mRNA/DOTMA LPNPs prepared with luciferase mRNA as a model also exhibit efficient protein expression performance at the animal level.

### Example 1.3: mRNA/MC3 LPNPs (unsaturated double bond ionizable lipid-LPNPs)

This example differs from Example 1.1 only in that the cationic lipids in Example 1.1 were replaced with MC3. The rest of the preparation process remained the same to obtain mRNA/MC3 LPNPs, which were also subjected to the following structure and performance characterization.

### 1. Size and morphology of mRNA/MC3 LPNPs

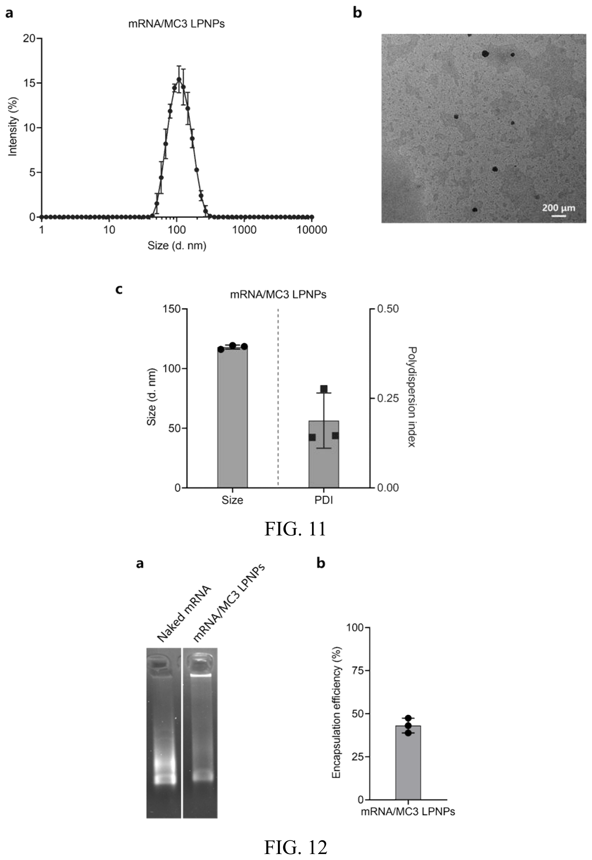

FIG. 11 shows the characterization results of the size and morphology of mRNA/MC3 LPNPs. FIG. 11-a shows DLS size distribution. The DLS results show uniform size distribution (d.size = 118 ± 2 nm and PDI = 0.19 ± 0.08). FIG. 11-b shows a TEM image showing that the prepared mRNA nanoparticles have a quasi-spherical structure. FIG. 11-c shows the sizes and PDI distribution of different preparation batches.

### 2. mRNA encapsulation efficiency of mRNA/MC3 LPNPs

FIG. 12 shows the characterization of mRNA encapsulation efficiency of mRNA/MC3 LPNPs. FIG. 12-a shows a representative agarose gel electrophoresis image of mRNA/MC3 LPNPs. FIG. 12-b shows the mRNA encapsulation efficiency of mRNA/MC3 LPNPs prepared in different preparation batches. FIG. 12 shows that the prepared mRNA/MC3 LPNPs can achieve an mRNA encapsulation efficiency of approximately 43%.

### 3. Size stability of mRNA/MC3 LPNPs

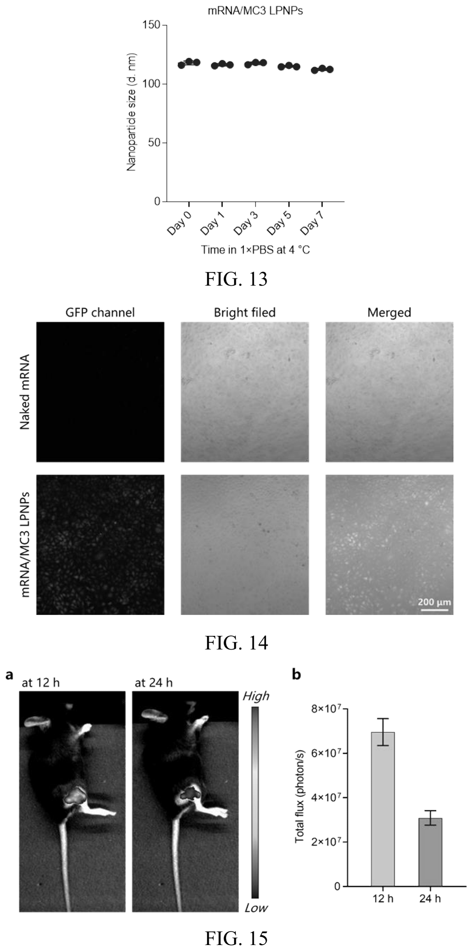

FIG. 13 shows variations of sizes of mRNA/MC3 LPNPs with time during storage in 1× PBS at 4°C. FIG. 13 shows that the prepared mRNA/MC3 LPNPs have good size stability in 1× PBS at 4°C.

### 4. GFP expression of mRNA/MC3 LPNPs at the cell level

FIG. 14 shows GFP fluorescence images of A549 cells co-incubated with mRNA/MC3 LPNPs (1.0 ng/µL mRNA) for 24 h. FIG. 14 shows that the mRNA/MC3 LPNPs prepared with GFP mRNA as a model achieve protein expression at the cell level.

### 5. Luciferase expression of mRNA/MC3 LPNPs at the animal level

FIG. 15 shows luciferase expression levels at different time points after intramuscular injection of mRNA/MC3 LPNPs (50 ng/µL mRNA, 50 µL). FIG. 15 shows that the mRNA/MC3 LPNPs prepared with luciferase mRNA as a model can also achieve protein expression at the animal level.

### Example 1.4: mRNA/C12-200 LPNPs (multi-tailed ionizable lipid-LPNPs)

This example differs from Example 1.1 only in that the cationic lipids in Example 1.1 were replaced with C12-200. The rest of the preparation process remained the same to obtain mRNA/C12-200 LPNPs, which were also subjected to the following structure and performance characterization.

### 1. Size and morphology of mRNA/C12-200 LPNPs

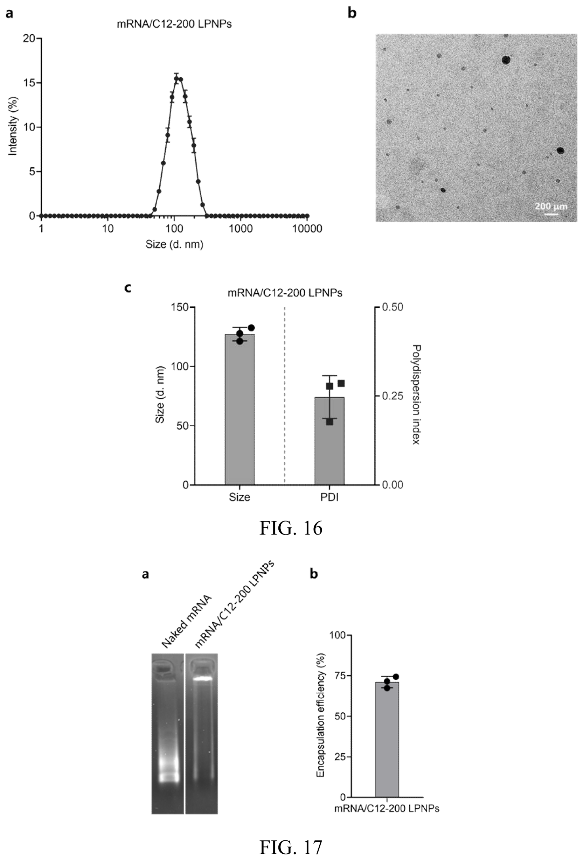

FIG. 16 shows the characterization results of the size and morphology of mRNA/C12-200 LPNPs. FIG. 16-a shows DLS size distribution. The DLS results show uniform size distribution (d.size = 128 ± 6 nm and PDI = 0.25 ± 0.06). FIG. 16-b shows a TEM image showing that the prepared mRNA nanoparticles have a quasi-spherical structure. FIG. 16-c shows the sizes and PDI distribution of different preparation batches.

### 2. mRNA encapsulation efficiency of mRNA/C12-200 LPNPs

FIG. 17 shows the characterization of mRNA encapsulation efficiency of mRNA/C12-200 LPNPs. FIG. 17-a shows a representative agarose gel electrophoresis image of mRNA/C12-200 LPNPs. FIG. 17-b shows the mRNA encapsulation efficiency of mRNA/C12-200 LPNPs prepared in different preparation batches. FIG. 17 shows that the prepared mRNA/C12-200 LPNPs can achieve an mRNA encapsulation efficiency of approximately 71%.

### 3. Size stability of mRNA/C12-200 LPNPs

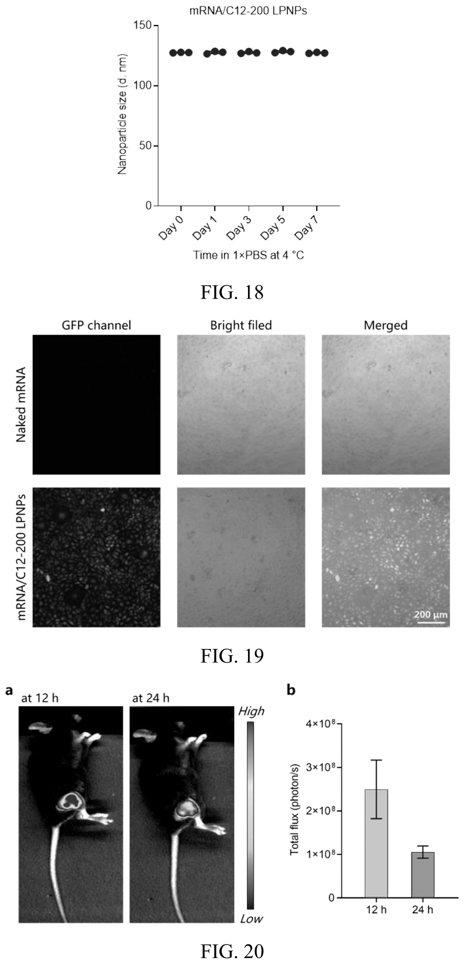

FIG. 18 shows variations of sizes of mRNA/C12-200 LPNPs with time during storage in 1× PBS at 4°C. FIG. 18 shows that the prepared mRNA/C12-200 LPNPs have good size stability in 1× PBS at 4°C.

### 4. GFP expression of mRNA/C 12-200 LPNPs at the cell level

FIG. 19 shows GFP fluorescence images of A549 cells co-incubated with mRNA/C12-200 LPNPs (1.0 ng/µL mRNA) for 24 h. FIG. 19 shows that the mRNA/C12-200 LPNPs prepared with GFP mRNA as a model exhibit efficient protein expression performance at the cell level.

### 5. Luciferase expression of mRNA/C12-200 LPNPs at the animal level

FIG. 20 shows luciferase expression levels at different time points after intramuscular injection of mRNA/C12-200 LPNPs (50 ng/µL mRNA, 50 µL). FIG. 20-a shows the bioluminescence images of a mouse at different time points after intramuscular injection of mRNA/C12-200 LPNPs. FIG. 20-b shows a quantitative analysis of bioluminescence of mice. FIG. 20 shows that the mRNA/C12-200 LPNPs prepared with luciferase mRNA as a model also exhibit efficient protein expression performance at the animal level.

### Example 1.5: mRNA/SM-102 LPNPs (multi-tailed ionizable lipid-LPNPs (ionizable lipid from Moderna))

This example differs from Example 1.1 only in that the cationic lipids in Example 1.1 were replaced with SM-102. The rest of the preparation process remained the same to obtain mRNA/SM-102 LPNPs, which were also subjected to the following structure and performance characterization.

### 1. Size and morphology of mRNA/SM-102 LPNPs

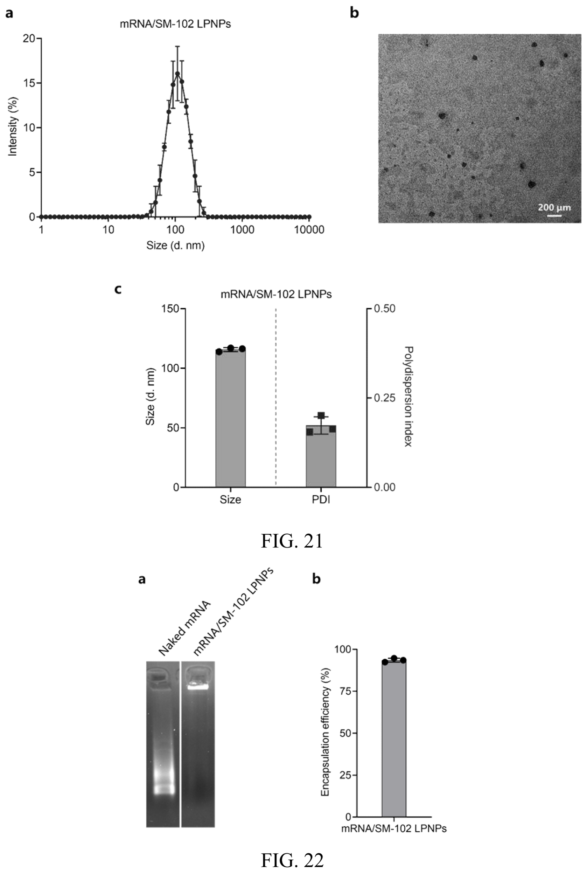

FIG. 21 shows the characterization results of the size and morphology of mRNA/SM-102 LPNPs. FIG. 21-a shows DLS size distribution. The DLS results show uniform size distribution (d.size = 116 ± 2 nm and PDI = 0.17 ± 0.02). FIG. 21-b shows a TEM image showing that the prepared mRNA nanoparticles have a quasi-spherical structure. FIG. 21-c shows the sizes and PDI distribution of different preparation batches.

### 2. mRNA encapsulation efficiency of mRNA/SM-102 LPNPs

FIG. 22 shows the characterization of mRNA encapsulation efficiency of mRNA/SM-102 LPNPs. FIG. 22-a shows a representative agarose gel electrophoresis image of mRNA/SM-102 LPNPs. FIG. 22-b shows the mRNA encapsulation efficiency of mRNA/SM-102 LPNPs prepared in different preparation batches. FIG. 22 shows that the prepared mRNA/SM-102 LPNPs can almost achieve complete mRNA encapsulation, with an encapsulation efficiency of approximately 94%.

### 3. Size stability of mRNA/SM-102 LPNPs

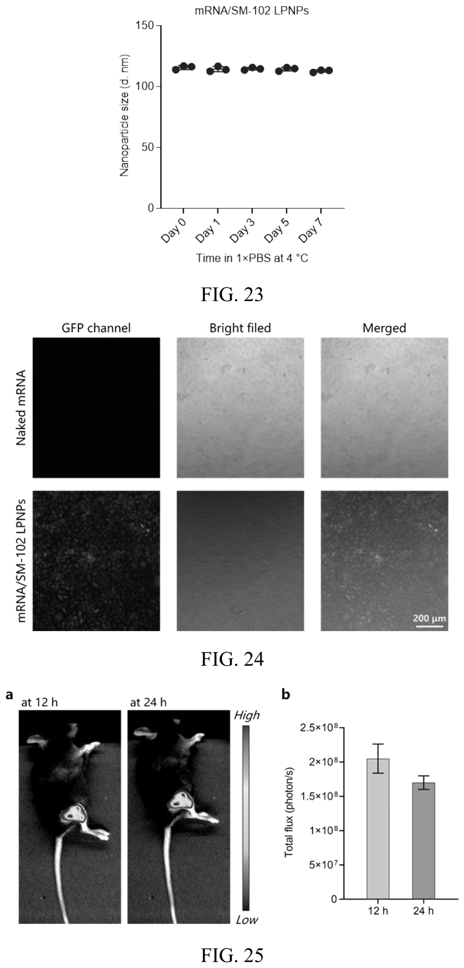

FIG. 23 shows variations of sizes of mRNA/SM-102 LPNPs with time during storage in 1× PBS at 4°C. FIG. 23 shows that the prepared mRNA/SM-102 LPNPs have good size stability in 1× PBS at 4°C.

### 4. GFP expression of mRNA/SM-102 LPNPs at the cell level

FIG. 24 shows GFP fluorescence images of A549 cells co-incubated with mRNA/SM-102 LPNPs (1.0 ng/µL mRNA) for 24 h. FIG. 24 shows that the mRNA/SM-102 LPNPs prepared with GFP mRNA as a model exhibit efficient protein expression performance at the cell level.

### 5. Luciferase expression of mRNA/SM-102 LPNPs at the animal level

FIG. 25 shows luciferase expression levels at different time points after intramuscular injection of mRNA/SM-102 LPNPs (50 ng/µL mRNA, 50 µL). FIG. 25-a shows the bioluminescence images of a mouse at different time points after intramuscular injection of mRNA/SM-102 LPNPs. FIG. 25-b shows a quantitative analysis of bioluminescence of mice. FIG. 25 shows that the mRNA/SM-102 LPNPs prepared with luciferase mRNA as a model also exhibit efficient protein expression performance at the animal level.

### Example 2: Preparation of mRNA PDSA LPNPs (polymer PDSA-based mRNA-LPNPs)

With PDSA as the polymer, G0-C14 as the cationic lipid, and DSPE-PEG (CAS No.: 147867-65-0) as the modified amphiphilic molecule in the mRNA-LPNPs delivery system, mRNA PDSA LPNPs were prepared by the specific steps below.
(1) The hydrophobic polymer PDSA was synthesized through one-step polycondensation of (H-Cys-OMe)₂-2HCl and a fatty acid dichloride and characterized through proton nuclear magnetic resonance (¹H NMR).
(2) Preparation of key component stock solutions: G0-C14, PDSA, and DSPE-PEG were fully dissolved in DMF, whose concentrations were 2.5 mg/mL, 20 mg/mL, and 20 mg/mL, respectively.
(3) 24 µg of mRNA (1.0 µg/µL) was mixed with 360 µg of G0-C14 (144 µL of G0-C14-containing solution prepared in step (2)) to obtain a G0-C14/mRNA electrostatic complex.
(4) 4 mg of PDSA (200 µL of PDSA-containing solution prepared in step (2)) and 2.8 mg of DSPE-PEG (140 µL of DSPE-PEG-containing solution prepared in step (2)) were added sequentially to the above mixture to obtain solution A.
(5) Under magnetic stirring (800 rpm), solution A was added dropwise to 10 mL of ultrapure water and continued being stirred for 30 min.
(6) An organic solvent was removed using an ultrafiltration centrifugation method (molecular weight cutoff: 100 kDa), which was repeated three times under the centrifugation conditions of 3000 g and 8 min.

Finally, the volume was adjusted to 600 µL with 1×PBS to obtain mRNA/G0-C14 LPNPs (mRNA concentration: 40 ng/µL).

### Structure and performance characterization

### 1. Morphology, size, and stability of mRNA PDSA LPNPs

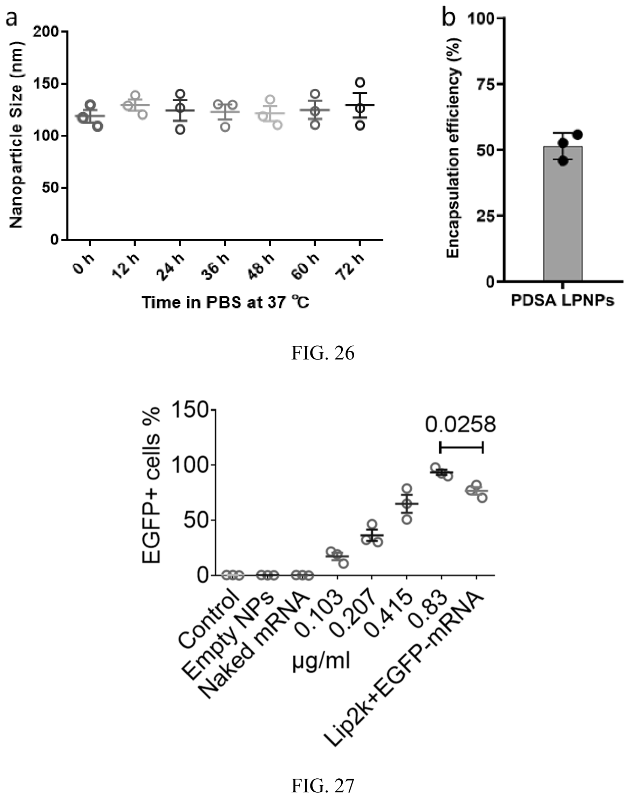

FIG. 26 shows the size, stability, and encapsulation efficiency of mRNA PDSA LPNPs. FIG. 26-a shows variations of sizes of mRNA PDSA LPNPs in PBS containing 10% fetal bovine serum. FIG. 26-a shows that the prepared mRNA PDSA LPNPs have good size stability in serum-containing PBS.

### 2. mRNA encapsulation efficiency of mRNA PDSA LPNPs

The mRNA encapsulation efficiency was measured through fluorescence quantification. Specifically, Cy5-labeled mRNA was quantified via fluorescence to determine the mRNA content. A fluorescence intensity of Cy5-mRNA encapsulated in the mRNA nanoparticles was measured using a microplate reader (TECAN, Infinite M200 Pro). The measured mRNA encapsulation efficiency of the synthesized mRNA PDSA LPNPs was determined to be approximately 50%, as shown in FIG. 26-b.

### 3. EGFP expression of mRNA PDSA LPNPs at the cell level

FIG. 27 shows the characterization of an EGFP expression capability of mRNA PDSA LPNPs in Hep3B cells. FIG. 27 shows the EGFP positive rates of Hep3B cells co-incubated with mRNA nanoparticles having different concentrations. FIG. 27 shows that the mRNA PDSA LPNPs prepared with EGFP mRNA as a model exhibit efficient protein expression performance at the cell level.

### 4. Cytotoxicity evaluation of mRNA PDSA LPNPs

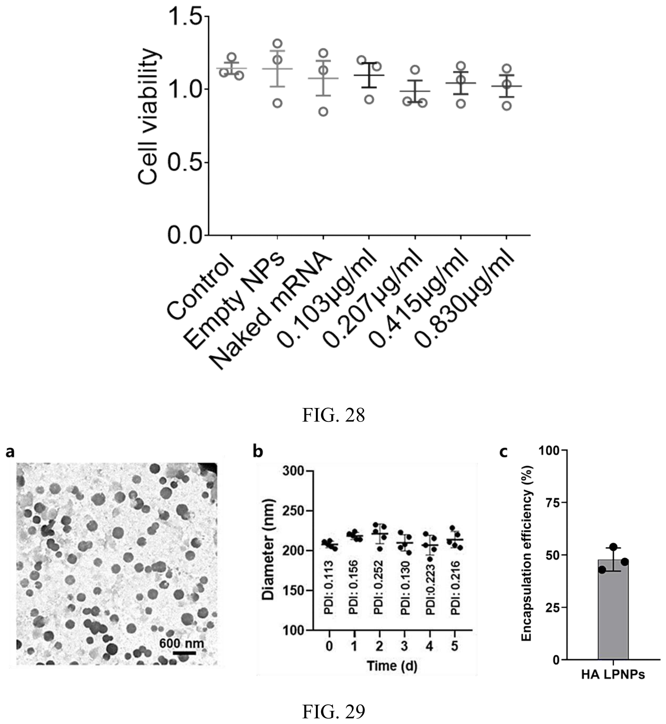

FIG. 28 shows the cell viability of Hep3B cells co-incubated with mRNA PDSA LPNPs having different concentrations. FIG. 28 shows that EGFP mRNA PDSA LPNPs exhibit good cell safety.

### Example 3: Preparation of mRNA HA LPNPs (polymer HA-based mRNA-LPNPs)

With hyaluronic acid HA (having a molecular weight of 1.01 MDa to 1.80 MDa) as the polymer, G0-C14 as the cationic lipid, and DSPE-PEG (CAS No.: 147867-65-0) as the modified amphiphilic molecule in the mRNA-LPNPs delivery system, mRNA HA LPNPs were prepared by the specific steps below.
(1) Preparation of key component stock solutions: G0-C14, HA, and DSPE-PEG were fully dissolved in DMF, ultrapure water, and ultrapure water, respectively, whose concentrations were 0.5 mg/mL, 0.5 mg/mL, and 5.0 mg/mL, respectively.
(2) 20 µg of mRNA (1.0 µg/µL) was mixed with 250 µg of G0-C14 (500 µL) to obtain a G0-C14/mRNA electrostatic complex.
(3) 250 µg of HA (500 µL) was quickly added to the above electrostatic complex and mixed uniformly to obtain solution A.
(4) Under magnetic stirring (1200 rpm), solution A was added dropwise to 10 mL of ultrapure water containing 1 mg of DSPE-PEG and continued being stirred for 30 min.
(5) An organic solvent was removed through ultrafiltration centrifugation (molecular weight cutoff: 100 kDa), which was repeated three times under the centrifugation conditions of 3000 g and 8 min.
(6) Finally, the volume was adjusted to 500 µL with 1×PBS to obtain mRNA/G0-C14 LPNPs (mRNA concentration: 40 ng/µL).

### Structure and performance characterization

### 1. Morphology, size, and stability of mRNA HA LPNPs

FIG. 29 shows the characterization of the morphology, size, and stability and the encapsulation efficiency of mRNA HA LPNPs. FIG. 29-a shows a TEM image showing that the prepared mRNA nanoparticles have a quasi-spherical structure with a size of approximately 200 nm. FIG. 29-b shows variations of sizes of mRNA HA LPNPs in PBS. FIG. 29-b shows that the prepared mRNA HA LPNPs have good size stability during storage in PBS.

### 2. mRNA encapsulation efficiency of mRNA HA LPNPs

The mRNA encapsulation efficiency was measured through fluorescence quantification. Specifically, Cy5-labeled mRNA was quantified via fluorescence to determine the mRNA content. A fluorescence intensity of Cy5-mRNA encapsulated in the mRNA nanoparticles was measured using a microplate reader (TECAN, Infinite M200 Pro). The measured mRNA encapsulation efficiency of the synthesized mRNA HA LPNPs was determined to be approximately 50%, as shown in FIG. 29-c.

### 3. EGFP expression of mRNA HA LPNPs at the cell level

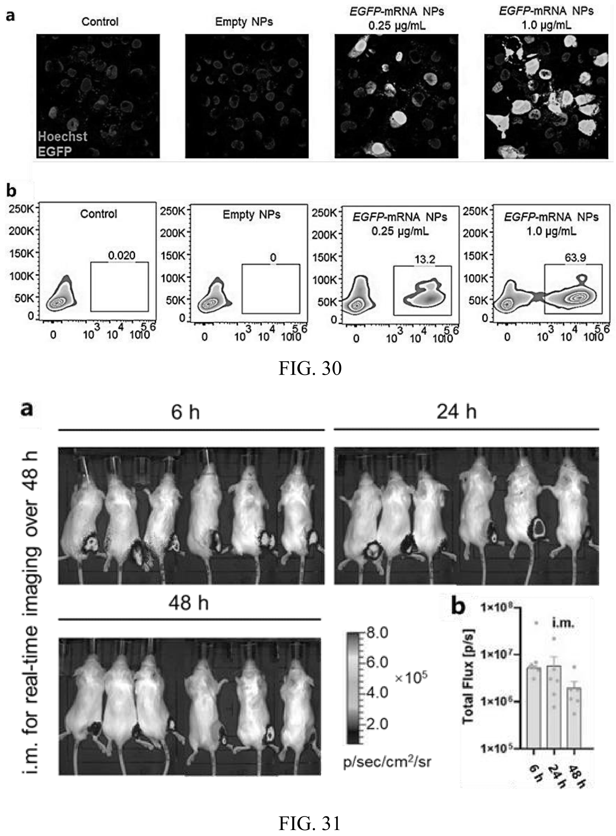

FIG. 30 shows the characterization of an EGFP expression capability of mRNA HA LPNPs in H1299 cells. FIG. 30-a shows EGFP fluorescence images after co-incubation with H1299 cells for 24 h. FIG. 30-b shows the flow cytometry characterization of H1299 cells co-incubated with different mRNA nanoparticles. FIG. 30 shows that the mRNA HA LPNPs prepared with EGFP mRNA as a model exhibit efficient protein expression performance at the cell level.

### 4. Luciferase expression of mRNA HA LPNPs at the animal level

FIG. 31 shows luciferase expression levels at different time points after intramuscular injection of mRNA HA LPNPs (50 ng/µL mRNA, 50 µL). FIG. 31-a shows the bioluminescence images of mice at different time points after intramuscular injection of mRNA HA LPNPs. FIG. 31-b shows a quantitative analysis of bioluminescence of mice. FIG. 31 shows that the mRNA HA LPNPs prepared with luciferase mRNA as a model also exhibit efficient protein expression performance at the animal level.

### Example 4: Microfluidic preparation of mRNA/G0-C14 LPNPs

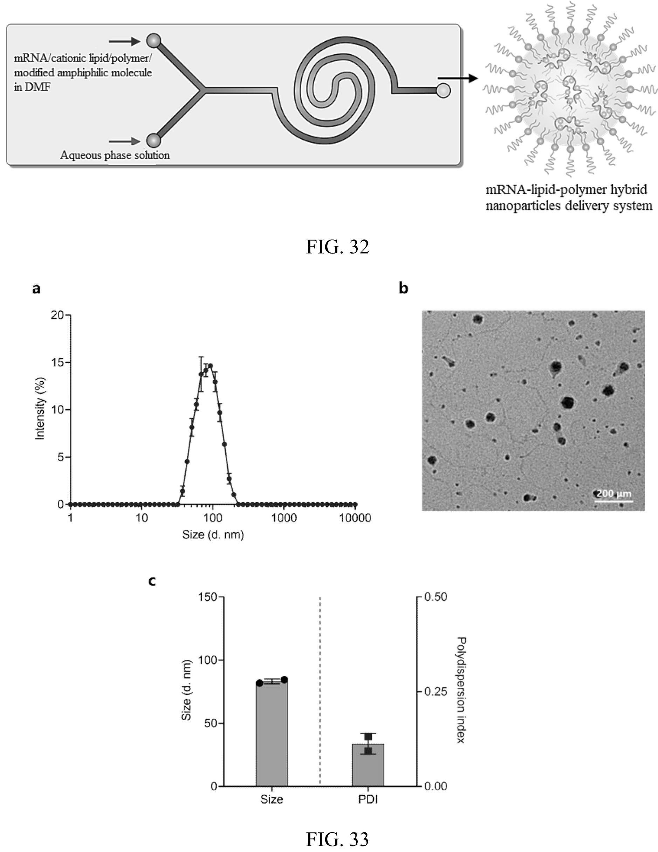

The present application provides a method for preparing mRNA-LPNPs through dual-channel microfluidics. mRNA and cationic lipids formed a complex, a polymer and modified amphiphilic molecules were sequentially added to the mixed solution and mixed into a uniform DMF organic phase solution. Two phases were mixed using a dual-channel microfluidic device (as shown in FIG. 32). The effluent was subjected to ultrafiltration to remove an organic solvent to obtain mRNA-LPNPs. Microfluidic operation conditions were as follows: a flowrate of the DMF organic phase was 2 mL/min, a flowrate of an aqueous phase solution was 5 mL/min, and a synthesis volume was 500 µL to 20 mL.

With "mRNA/G0-C14 LPNPs" as an example, the microfluidic preparation of mRNA/G0-C14 LPNPs includes the specific steps below.
(1) Preparation of key component stock solutions: G0-C14, PLGA, and DSPE-PEG were fully dissolved in DMF to obtain the corresponding stock solutions: G0-C14 (2.5 mg/mL), PLGA (5.0 mg/mL), and DSPE-PEG (20 mg/mL).
(2) 50 mM and 10 mM citrate buffer solutions (pH = 4.0) were prepared and treated with DEPC to be made nuclease-free.
(3) Preparation of an mRNA/G0-C14 complex: 50 µL of mRNA (1 µg/µL) was taken, added with 12.5 µL of citrate buffer (50 mM), mixed uniformly, quickly added to 250 µL of G0-C14 DMF solution under vortexing, and continued being vortexed for 20s to obtain the mRNA/G0-C14 complex.
(4) 250 µL of PLGA DMF solution and 125 µL of DSPE-PEG DMF solution were sequentially added to the above mRNA/G0-C14 complex and mixed uniformly to obtain the DMF organic phase.
(5) The aqueous phase solution was 3125 µL of citrate buffer (10 mM).
(6) Under microfluidic (Y-shaped chip/Micro&Nano) conditions, mRNA-LPNPs were prepared with the flowrate of the DMF organic phase being 2 mL/min and the flowrate of the aqueous phase solution being 5 mL/min.
(7) Under vortexing, 3125 µL of 1× PBS was slowly added dropwise to the above mixed solution.
(8) An organic solvent was removed through ultrafiltration centrifugation (molecular weight cutoff: 100 kDa), which was repeated three times under the centrifugation conditions of 3000 g and 8 min.
(9) Finally, the volume was adjusted to 1000 µL with 1×PBS to obtain mRNA/G0-C14 LPNPs (mRNA concentration: 50 ng/µL).

### Structure and performance characterization

### 1. Size and morphology characterization of mRNA/G0-C14 LPNPs prepared through microfluidics

FIG. 33 shows the size and morphology characterization of mRNA/G0-C14 LPNPs prepared through microfluidics. FIG. 33-a shows DLS size distribution. FIG. 33-b shows a TEM image. FIG. 33-c shows the sizes and PDI distribution of different preparation batches. FIG. 33 shows that the mRNA/G0-C14 LPNPs prepared through microfluidics have a size of approximately 89 nm and a PDI of approximately 0.114, exhibiting a smaller size and lower polydispersity.

### 2. mRNA encapsulation efficiency of mRNA/G0-C14 LPNPs prepared through microfluidics

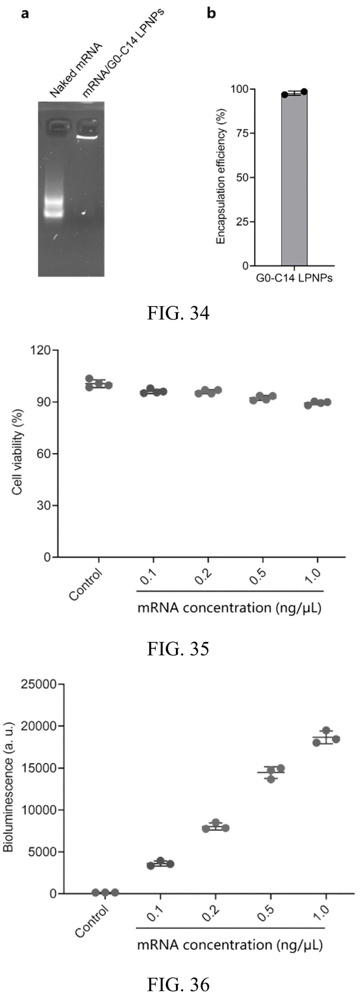

FIG. 34 shows the characterization of mRNA encapsulation efficiency of mRNA/G0-C14 LPNPs prepared through microfluidics. FIG. 34-a shows a representative agarose gel electrophoresis image of mRNA/G0-C14 LPNPs prepared through microfluidics. FIG. 34-b shows the mRNA encapsulation efficiency of mRNA/G0-C14 LPNPs prepared through microfluidics in different preparation batches. FIG. 34 shows that the mRNA/G0-C14 LPNPs prepared through microfluidics can almost achieve complete mRNA encapsulation, with an encapsulation efficiency of approximately 100%.

### 3. Cytotoxicity evaluation of mRNA/G0-C14 LPNPs

FIG. 35 shows the *in vitro* cytotoxicity of mRNA/G0-C14 LPNPs having different concentrations. FIG. 35 shows that eGFP mRNA/G0-C14 LPNPs exhibit good cell safety.

### 4. Luci-mRNA expression capability of mRNA/G0-C14 LPNPs prepared through microfluidics

FIG. 36 shows the bioluminescence levels of A549 cells transfected with mRNA/G0-C14 LPNPs having different concentrations. FIG. 37 shows luciferase expression levels at different time points after intramuscular injection of mRNA/G0-C14 LPNPs (50 ng/µL mRNA, 50 µL). FIG. 37-a shows the bioluminescence images of a mouse at different time points after intramuscular injection of mRNA/G0-C14 LPNPs. FIG. 37-b shows a quantitative analysis of bioluminescence of mice.

FIGS. 36 and 37 show that the mRNA LPNPs prepared through microfluidics exhibit efficient protein expression capabilities at both the cell level and the animal level.

The inventors of the present application have also conducted experiments using other materials, process operations, and process conditions described in the specification with reference to the preceding embodiments and obtained relatively satisfactory results.

Aspects, embodiments, features, and examples of the present application are illustrative in all respects and are not intended to limit the present application. The scope of the present application is defined by the appended claims. Other embodiments, modifications, and uses are to be understood by those skilled in the art without departing from the spirit and scope of the present application.

The use of titles and sections in the present application does not imply any limitation on the present application; each section can be applied to any aspect, embodiment, or feature of the present application.

Throughout the present application, where a composition is described as having, comprising, or including particular components or where a process is described as having or including particular process steps, it means that the composition taught in the present application consists substantially of or consists of the described components and that the process taught in the present application consists substantially of or consists of the described process steps.

It is to be understood that the order of steps or the execution sequence of particular actions is not critical so long as the teachings of the present application remain operable. Additionally, two or more steps or actions may be performed simultaneously.

Preferred embodiments of the present application are described above and are not intended to impose any formal or substantive limitation on the present application. Those skilled in the art can make some equivalent changes such as variations, modifications, and evolutions based on the technical content disclosed above without departing from the scope of the technical solutions of the present application, and these equivalent changes are all equivalent embodiments of the present application. Meanwhile, any equivalent changes such as variations, modifications, and evolutions made to the preceding embodiments based on the substantive techniques of the present application are within the scope of the technical solutions of the present application.

## Claims

1. An mRNA-lipid-polymer hybrid nanoparticles delivery system, comprising mRNA, a cationic molecule, a polymer, and a modified amphiphilic molecule as components, wherein the cationic molecule and negatively charged mRNA form a complex through electrostatic interaction, the polymer is used to encapsulate the cationic molecule-mRNA complex to form a stable core structure, and the modified amphiphilic molecule is anchored to a surface of the core structure through hydrophobic interaction to form a quasi-spherical core-shell structure.

2. The mRNA-lipid-polymer hybrid nanoparticles delivery system according to claim 1, wherein mRNA is selected from at least one of self-reporter gene mRNA, genome editing mRNA, antigen mRNA, tumor suppressor gene mRNA, interleukin mRNA, chimeric antigen receptor mRNA, or other functional mRNA.

3. The mRNA-lipid-polymer hybrid nanoparticles delivery system according to claim 2, wherein
the self-reporter gene mRNA is selected from one of GFP, eGFP, Fluc, mCherry, beta gal, or renilla Luc;
the genome editing mRNA is selected from one of Cas9, Cas9 Nickase, or Cre recombinase;
the antigen mRNA is selected from OVA;
the tumor suppressor gene mRNA is selected from one of p53 or PTEN; and
the interleukin mRNA is selected from one of IL-10, IL-12, or IL-6.

4. The mRNA-lipid-polymer hybrid nanoparticles delivery system according to claim 1, wherein the cationic molecule is selected from at least one of ammonia derivatives, ammonium salts and derivatives thereof, positively charged amphiphilic lipid-like compounds, polyamide and derivatives thereof, or polyethyleneimine and derivatives thereof.

5. The mRNA-lipid-polymer hybrid nanoparticles delivery system according to claim 1, wherein the polymer is selected from at least one of aliphatic polyester polymers and derivatives thereof, polycarbonates and derivatives thereof, polysaccharides and derivatives thereof, polyamino acids and derivatives thereof, polymeric polyols and derivatives thereof, or polyacrylic acids and derivatives thereof.

6. The mRNA-lipid-polymer hybrid nanoparticles delivery system according to claim 5, wherein the aliphatic polyester polymers and derivatives thereof are selected from one of polylactic acid, polycaprolactone, or poly(lactic-co-glycolic acid).

7. The mRNA-lipid-polymer hybrid nanoparticles delivery system according to claim 1, wherein the modified amphiphilic molecule is selected from at least one of a polyethylene glycolated amphiphilic molecule, an amphiphilic surfactant, an amphiphilic protein, or other modified molecules.

8. The mRNA-lipid-polymer hybrid nanoparticles delivery system according to claim 7, wherein
the polyethylene glycolated amphiphilic molecule is selected from one of DSPE-PEG, DMG-PEG, or F127;
the amphiphilic surfactant is CO-520;
the amphiphilic proteins are albumin; and
the other modified molecules are selected from one of mannitol, modified chitosan, dextran, carboxydextran, tetraethyl orthosilicate, polyacrylic acid, KH560, KH550, diethylenetriaminepentaacetic acid, meglumine, arginine, polyglutamic acid, or polypeptides.

9. A preparation method for an mRNA-lipid-polymer hybrid nanoparticle delivery system, comprising:
(a) preparing a solution of a cationic molecule;
(b) forming an electrostatic complex of mRNA and the cationic molecule;
(c) sequentially adding a polymer and a modified amphiphilic molecule to an mRNA/cationic molecule complex formed above; and
(d) obtaining stable mRNA-encapsulated nanoparticles by a self-assembly method, that is, the mRNA-lipid-polymer hybrid nanoparticles delivery system delivery system.

10. The preparation method according to claim 9, comprising specific steps of:
(a1) dissolving a cationic molecule in a solvent to prepare a solution containing the cationic molecule and having a concentration of 0.5-5.0 mg/mL;
wherein the cationic molecule is selected from at least one of ammonia derivatives, ammonium salts and derivatives thereof, positively charged amphiphilic lipid-like compounds, polyamide and derivatives thereof, or polyethyleneimine and derivatives thereof; and
the solvent is selected from at least one of methanol, ethanol, ethylene glycol, diethylene glycol, DMF, benzyl alcohol, acetone, dichloromethane, or trichloromethane;
(b1) mixing a solution containing mRNA with the solution containing the cationic molecule uniformly, and performing a reaction for a period to obtain an mRNA/cationic molecule electrostatic complex;
wherein the reaction is performed for a period of 5s to 30 min and at a temperature of 0°C to 37°C;
a concentration of mRNA in the solution containing mRNA is 0.1-10 mg/mL;
a mass ratio of mRNA to the cationic molecule is 1:1 to 1:100;
mRNA is selected from at least one of self-reporter gene mRNA, genome editing mRNA, antigen mRNA, tumor suppressor gene mRNA, interleukin mRNA, chimeric antigen receptor mRNA, or other functional mRNA;
the self-reporter gene mRNA is selected from one of GFP, eGFP, Fluc, mCherry, beta gal, or renilla Luc;
the genome editing mRNA is selected from one of Cas9, Cas9 Nickase, or Cre recombinase;
the antigen mRNA is selected from OVA;
the tumor suppressor gene mRNA is selected from one of p53 or PTEN; and
the interleukin mRNA is selected from one of IL-10, IL-12, or IL-6;
(c1) mixing the mRNA/cationic molecule electrostatic complex with a polymer solution uniformly;
wherein a reaction is performed for a period of 5s to 30 min and at a temperature of 0°C to 37°C;
a concentration of the polymer molecule in a solution containing the polymer molecule is 0.1-20 mg/mL; and
the polymer is selected from at least one of aliphatic polyester polymers and derivatives thereof, polycarbonates and derivatives thereof, polysaccharides and derivatives thereof, polyamino acids and derivatives thereof, polymeric polyols and derivatives thereof, or polyacrylic acids and derivatives thereof;
(d1) adding a solution of the modified amphiphilic molecule into an mRNA/cationic molecule/polymer mixture solution and mixing the solutions uniformly;
wherein a concentration of the modified amphiphilic molecules in the solution of the modified amphiphilic molecule is 0.1-20 mg/mL; and
the modified amphiphilic molecule is selected from at least one of polyethylene glycolated amphiphilic molecules, an amphiphilic surfactant, amphiphilic proteins, or other modified molecules;
(e1) adding an mRNA/cationic molecule/polymer/modified amphiphilic molecule mixture solution into an acidic aqueous phase solution and performing a reaction for a period to obtain a particle of the mRNA-LPNPs nano-delivery system;
wherein the acidic solution has a pH range of 1-6;
the reaction is performed for a period of 30 s to 30 min and at a temperature of 0°C to 37°C;
the acidic buffer solution comprises, but is not limited to, at least one of citric acid/citrate, acetic acid/acetate, hydrochloric acid/hydrochloride, sulfuric acid/sulfate, or nitric acid/nitrate; and
in (b1), (c1), (d1), and (e1), a solution mixing manner comprises, but is not limited to, at least one of dropwise addition, stirring, standing, a microfluidic device, a peristaltic pump, or a microinjection pump.

11. The preparation method according to claim 10, wherein the aliphatic polyester polymers and derivatives thereof in (c1) are selected from one of polylactic acid, polycaprolactone, or poly(lactic-co-glycolic acid).

12. The preparation method according to claim 10, wherein in (d1), the polyethylene glycolated amphiphilic molecules are selected from one of DSPE-PEG, DMG-PEG, or F127;
the amphiphilic surfactant is CO-520;
the amphiphilic proteins are albumin; and
the other modified molecules are selected from one of mannitol, modified chitosan, dextran, carboxydextran, tetraethyl orthosilicate, polyacrylic acid, KH560, KH550, diethylenetriaminepentaacetic acid, meglumine, arginine, polyglutamic acid, or polypeptides.

13. The preparation method according to any one of claims 10 to 12, wherein the preparation method adopts the microfluidic device and comprises (a1), (b1), and (c1);
(d1) adding the solution of the modified amphiphilic molecules into the mRNA/cationic molecule/polymer mixture solution prepared in (c1) and mixing the solutions uniformly to obtain DMF organic phases;
(e1) preparing an aqueous phase solution; and
(f1) under microfluidic conditions, preparing mRNA-LPNPs with a flowrate of the DMF organic phases being 1-5 mL/min and a flowrate of the aqueous phase solution being 5-25 mL/min.

14. An mRNA-lipid-polymer hybrid nanoparticles delivery system prepared by the preparation method according to any one of claims 9 to 13.

15. The mRNA-lipid-polymer hybrid nanoparticles delivery system according to any one of claims 1 to 8 or 14, wherein encapsulation efficiency of mRNA in the mRNA-LPNPs delivery system is ≥ 50%, preferably ≥ 60%, more preferably ≥ 70%, more preferably ≥ 80%, more preferably ≥ 90%, and most preferably ≥ 95%.

16. The mRNA-lipid-polymer hybrid nanoparticles delivery system according to any one of claims 1 to 8 or 14, wherein a mass ratio of mRNA to other molecules in the mRNA-LPNPs delivery system is 1:100 to 10:1.

17. The mRNA-lipid-polymer hybrid nanoparticles delivery system according to any one of claims 1 to 8 or 14, wherein the mRNA-LPNPs delivery system has a two-dimensional, three-dimensional, or layered structure; the particle of the nano-delivery system is a three-dimensional nanocomposite material with an mRNA-cationic molecule-polymer core coated with a shell of the modified amphiphilic molecules; and a particle size of the particle of the nano-delivery system is 10-500 nm, preferably 20-250 nm, more preferably 30-200 nm, more preferably 70-130 nm, more preferably 100-125 nm, and most preferably 110-125 nm.

18. The mRNA-lipid-polymer hybrid nanoparticles delivery system according to any one of claims 1 to 8 or 14, wherein based on a total number of mRNA-LPNPs delivery systems, 80%, preferably 85%, more preferably 90%, more preferably 95%, and most preferably ≥ 99% of the delivery systems have particle sizes within ±15%, preferably ±10%, and more preferably ±1% of a D50 range of the nanocomposite material.

19. The mRNA-lipid-polymer hybrid nanoparticles delivery system according to any one of claims 1 to 8 or 14, wherein the mRNA-LPNPs delivery system is stably dispersible in an aqueous solution and 0.9% normal saline.

20. The mRNA-lipid-polymer hybrid nanoparticles delivery system according to any one of claims 1 to 8 or 14, wherein when the mRNA-LPNPs delivery system is stored in water or 0.9% normal saline at 4°C, 25°C, 37°C, -20°C, or -80°C for 5-20 days, preferably 20-40 days, and more preferably 40-60 days, an average particle size of the particle of the nano-delivery system changes by ≤ 20%, preferably ≤ 10%, more preferably ≤ 5%, and most preferably ≤ 3%.

21. The mRNA-lipid-polymer hybrid nanoparticle delivery system according to any one of claims 1 to 8 or 14, wherein cell transfection efficiency of mRNA in the mRNA-LPNPs delivery system is ≥ 50%, preferably ≥ 60%, more preferably ≥ 70%, more preferably ≥ 80%, more preferably ≥ 90%, and most preferably ≥ 95%.

22. Use of the mRNA-lipid-polymer hybrid nanoparticles delivery system according to any one of claims 1 to 8 or 14, wherein the mRNA-LPNPs delivery system is used for preparation of a tumor therapeutic drug, preparation of a tumor vaccine, preparation of a tumor-targeting drug, preparation of an *in vitro* or *in vivo* tumor diagnostic material, preparation of an inflammation-related disease treatment, preparation of an infectious disease treatment, preparation of infectious disease prevention, preparation of a wound-healing treatment, preparation of a cardiovascular and cerebrovascular chronic disease treatment, preparation of an organ fibrosis treatment, preparation of a viral infection treatment, preparation of viral infection prevention, preparation of an *in vitro* or *in vivo* gene-editing therapy, preparation of a non-alcoholic fatty liver treatment, preparation of an intestinal disease treatment, preparation of a neoantigen vaccine, preparation of cell differentiation induction, preparation of protein-based cosmetics, preparation of a protein defect-related disease treatment, preparation of aging repair, preparation of anti-aging, or preparation of a drug carrier.

23. A product comprising the mRNA-lipid-polymer hybrid nanoparticles delivery system according to any one of claims 1 to 8 or 14.
